(19) Europäisches Patentamt
European Patent Office
Office européen des brevets

(11) Veröffentlichungsnummer: **0 269 985**
**A2**

(12) # EUROPÄISCHE PATENTANMELDUNG

(21) Anmeldenummer: 87117374.6

(22) Anmeldetag: 25.11.87

(51) Int. Cl.[4]: **C07C 103/44**, C07C 103/82, C07C 143/78, C07D 213/56, C07C 127/19, A61K 31/165, A61K 31/17, A61K 31/44

(30) Priorität: 28.11.86 DE 3640829

(43) Veröffentlichungstag der Anmeldung: 08.06.88 Patentblatt 88/23

(84) Benannte Vertragsstaaten: AT BE CH DE ES FR GB GR IT LI LU NL SE

(71) Anmelder: **BOEHRINGER INGELHEIM KG**
D-6507 Ingelheim am Rhein(DE)

(84) BE CH DE ES FR GR IT LI LU NL SE AT

(71) Anmelder: **Boehringer Ingelheim International G.m.b.H**
D-6507 Ingelheim am Rhein(DE)

(84) GB

(72) Erfinder: **Köppe, Herbert, Dr.**
Neuweg 72
D-6507 Ingelheim am Rhein(DE)
Erfinder: **Esser, Franz, Dr.**
Posener Strasse 30
D-6507 Ingelheim am Rhein(DE)
Erfinder: **Kobinger, Walter, Prof.**
Belghofergasse 27
A-1120 Wien(AT)
Erfinder: **Lillie, Mag, Dr.**
Hansi-Niese-Weg 12
A-1130 Wien(AT)

(54) **Neue 1-Aryloxy-3-amino-2-propanole, ihre Herstellung und Verwendung.**

(57) Verbindungen der Formel

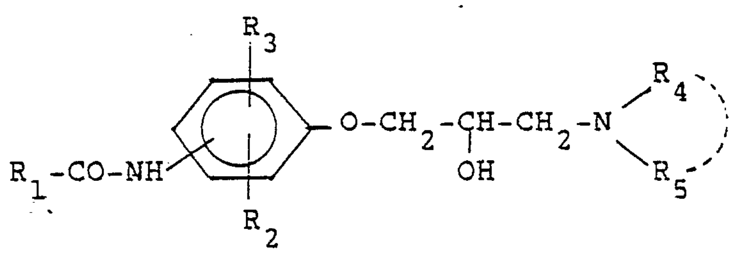

(I),

(die Substituenten $R_1$, $R_2$, $R_3$, $R_4$ und $R_5$ sind in der Beschreibung erklärt) können nach üblichen Methoden hergestellt werden.

EP 0 269 985 A2

Aufgrund eines günstigen Wirkungsprofils können die Verbindungen vorteilhaft zur Behandlung bestimmter Herz-und Kreislauferkrankungen eingesetzt werden.

Die Erfindung betrifft neue 1-Aryloxy-3-amino-2-propanole, ihre Herstellung in an sich bekannter Weise und ihre Verwendung als Arzneistoffe.

Die neuen Verbindungen entsprechen der Formel

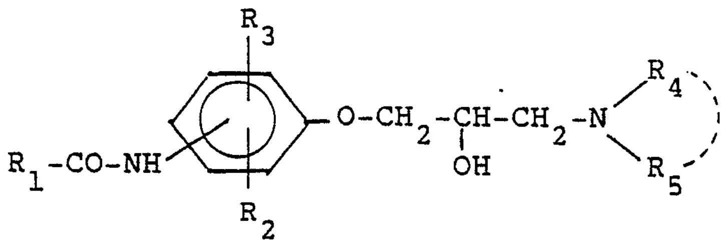

(I),

in der

$R_1$ einen Phenylrest, der gegebenenfalls durch ein oder mehrere Halogenatome, niedere Alkyl-, Alkoxy-, Alkenyl-, Alkinyl-, Alkenyloxy-, Alkinyloxy-, Cycloalkyl-, Acyl-, Acyloxy-, Alkoxycarbonyl-, Hydroxyalkyl- oder Alkoxyalkylreste oder die Sulfamoylgruppe oder die ringbildenden Gruppen $-(CH=CH)_2-$, $-O-CH_2-O-$, mit Bindung der freien Valenzen in o-Stellung zueinander, substituiert sein kann, oder einen Aryloxyalkylrest, der gegebenenfalls durch ein oder mehrere Halogenatome, niedere Alkyl-, Alkoxy-, Alkenyl-, Alkinyl-, Alkenyloxy-, Alkinyloxy, Hydroxyalkyl-, Alkoxyalkyl-, Acyl-, Acyloxy- oder Alkoxycarbonylreste sowie die ringbildende Gruppe $-(CH=CH)_2-$ oder $-OCH_2-O-$ mit Bindung der freien Valenzen in o-Stellung zueinander substituiert sein kann, oder einen Pyridylrest, oder einen Anilinorest, der durch ein oder mehrere Halogenatome oder niedere Alkylgruppen substituiert sein kann,

$R_2$ ein Wasserstoff- oder Halogenatom, eine Alkyl- oder Alkoxygruppe mit 1 bis 4 C-Atomen oder die

ringbildenden Gruppen $-(CH=CH)_2-$ oder $-(CH_2)_n-$ ($n$ = ganze Zahl von 3 bis 5) mit Bindung der freien Valenzen in o-Stellung zueinander, oder eine CN-Gruppe,

$R_3$ ein Wasserstoff- oder Halogenatom oder eine Alkylgruppe mit 1 bis 4 C-Atomen,

$R_4$ einen geraden oder verzweigten Alkylrest mit 1 bis 10 C-Atomen oder einen Hydroxyalkylrest mit 2 bis 5 C-Atomen,

$R_5$ einen geraden oder verzweigten Alkylrest mit 1 bis 10 C-Atomen oder einen Hydroxyalkylrest mit 2 bis 5 C-Atomen oder einen Phenylalkylrest oder einen Phenoxyalkylrest, wobei der aromatische Anteil durch Alkyl- oder Alkoxygruppen, durch Chlor- oder Bromatome substituiert sein kann,

$R_4$ und $R_5$ auch zusammen mit dem Stickstoffatom einem Heterocyclus, z.B. den Morpholin-, Piperidin- oder Piperazinring, bedeuten,

und können in Form von Racematen, reinen Enantiomeren oder Enantiomerengemischen sowie der jeweiligen Säureadditionssalze vorliegen.

Soweit nicht im einzelnen abweichende Angaben gemacht sind, werden die allgemeinen Definitionen im folgenden Sinn gebraucht.

Die "niederen" Alkyl-, Alkenyl-, Alkinyl-, Alkoxy-, Alkenyloxy-, Alkinyloxyreste enthalten bis zu 4 C-Atome, ebenso die Kohlenstoffketten in den Acyl-, Acyloxy-, Alkoxycarbonyl-, Hydroxyalkyl- oder Alkoxyalkylresten. Die Cycloalkylreste enthalten 3 bis 8, bevorzugt 5 bis 7

C-Atome. "Aryl" steht für Phenyl und Naphthyl, auch in Zusammensetzungen. "Halogen" bedeutet Chlor, daneben auch Brom und Fluor und in zweiter Linie Jod.

Reste der angegebenen Art sind demnach Methyl, Ethyl, n-Propyl, i-Propyl, die Butyle, Allyl, Propargyl und die jeweiligen -oxy-Gruppen, Acylgruppen wie $CH_3CO$, $C_2H_5-CO$ und entsprechende Acyloxygruppen, Alkoxycarbonylgruppen wie $COOC_2H_5$, $COOCH_3$, $COOC_3H_7$, Alkoxyalkylgruppen wie $C_2H_5OC_2H_4$, $H_3C-O-CH_2-CH_2-$, $C_3H_7OCH_2$, Cycloalkylgruppen wie Cyclopentyl, Cyclohexyl.

Bevorzugte Bedeutungen für $R_1$ sind substituierte Phenoxymethylreste.

Bevorzugte Bedeutungen für $R_2$ und $R_3$ sind Halogen und Methyl.

Bevorzugte Bedeutungen für $R_4$ und $R_5$ sind niedere gerade oder verzweigte Alkylreste, insbesondere der Methyl-, Ethyl-, n-Propyl- oder i-Propylrest.

Die neuen Verbindungen können in an sich bekannter Weise hergestellt werden durch

Umsetzung einer Verbindung der allgemeinen Formel

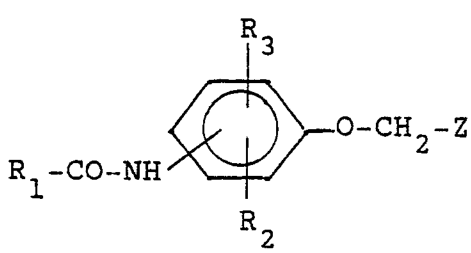

(II),

in der $R_1$, $R_2$ und $R_3$ wie in der Formel I definiert sind und Z die Gruppe 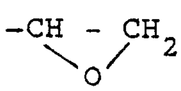 oder $-CHOH-CH_2-Hal$

(Hal = Halogen, z.B. Cl, Br) bedeutet, mit einem Amin der Formel

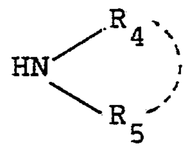

(III)

in der $R_4$ und $R_5$ die oben angegebene Bedeutung haben.

Soweit zunächst Säureadditionssalze erhalten werden, werden diese nach üblichen Methoden gewünschtenfalls in freie Basen oder in Salze anderer Säuren überführt. Zunächst erhaltene Basen werden gewünschtenfalls in Säureadditionssalze umgewandelt.

Das Verfahren wird vorzugsweise bei Temperaturen zwischen O und 100°C, insbesondere bei 40 bis 80°C durchgeführt.

Als Reaktionsmedien dienen Alkohole oder andere polare Lösungsmittel, z.B. Methanol, Ethanol, Isopropanol, Dioxan, Tetrahydrofuran, gegebenenfalls in Mischung.

Die Ausgangsverbindungen für das erfindungsgemäße Verfahren sind bekannt oder können nach bekannten Verfahren hergestellt werden.

Die erfindungsgemäßen Verbindungen besitzen mindestens ein asymmetrisches C-Atom (an der CHOH-Gruppe) und kommen daher als Racemat wie auch in Form der optischen Antipoden vor. Letztere können außer durch Racematentrennung mit üblichen Hilfssäuren wie Dibenzoyl- (bzw. Di-p-Toluyl-) D-Weinsäure oder D-3-Bromcampher-8-sulfonsäure oder auch durch Einsetzen von optisch aktivem Ausgangsmaterial erhalten werden.

Geeignete Säuren für die Herstellung physiologisch verträglicher Säureadditionssalze sind beispielsweise Salzsäure, Bromwasserstoffsäure, Schwefelsäure, Methansulfonsäure, Maleinsäure, Essigsäure, Oxalsäure, Milchsäure, Weinsäure oder 8-Chlortheophyllin.

Die neuen Verbindungen bzw. ihre physiologisch verträgliche Säureadditionssalze haben wertvolle therapeutische, insbesondere antiarrhythmische, blutdrucksenkende oder bradykarde Eigenschaften und können aufgrund ihres günstigen Wirkungsprofils beispielsweise vorteilhaft zur Behandlung von Herzarrhythmien, Tachykardien oder des Bluthochdrucks in der Humanmedizin eingesetzt werden.

Als wertvoll haben sich dabei insbesondere solche Verbindungen der allgemeinen Formel I herausgestellt, bei denen $R_1$ einen substituierten Phenoxymethylrest und $NR_4R_5$ einen Diethylaminorest, einen Methylethylaminorest oder einen Dimethylaminorest bedeutet (substituierte p-Phenoxyacetylaminophenoxy-3-diethyl-, -methylethyl- oder -dimethylamino-2-propanole). Besonders wertvoll sind insbesondere das 1-[2,6-Dimethyl-4-(2-(2,6-dimethyl- phenoxy)-acetylamino)-phenoxy]-2-hydroxy-3-diethylamino- propan und das 1-[2,6-Dimethyl-4-(2-m-tolyloxy- acetylamino)-phenoxy]-2-hydroxy-3-diethylamino-propan.

Die Einzeldosis der erfindungsgemäßen Substanzen liegt bei 1 bis 300 mg, vorzugsweise 10 bis 150 mg (oral) bzw. 1 bis 20 mg (parenteral).

Die erfindungsgemäßen Wirkstoffe können in die üblichen galenischen Anwendungsformen, wie Tabletten, Dragées,

Lösungen, Emulsionen, Pulver, Kapseln oder Depotformen gebracht werden, wobei zu deren Herstellung die üblichen pharmazeutischen Hilfsstoffe sowie die üblichen Fertigungsmethoden herangezogen werden können. Entsprechende Tabletten können beispielsweise durch Mischen der Wirkstoffe mit bekannten Hilfsstoffen, beispielsweise inerten Verdünnungsmitteln, wie Calciumcarbonat, Calciumphosphat oder Milchzucker, Sprengmitteln, wie Maisstärke oder Alginsäure, Bindemitteln, wie Stärke oder Gelatine, Schmiermitteln, wie Magnesiumstearat oder Talk, und/oder Mitteln zur Erzielung eines Depoteffekts, wie Carboxymethylcellulose, Celluloseacetphthalat, oder Polyvinylacetat erhalten werden.

Die Tabletten können auch aus mehreren Schichten bestehen. Entsprechend können Dragées durch Überziehen von analog den Tabletten hergestellten Kernen mit üblicherweise in Drageeüberzügen verwendeten Mitteln, beispielsweise Kollidon oder Schellack, Gummi arabicum, Talk, Titandioxid oder Zucker, hergestellt werden. Zur Erzielung eines Depoteffekts oder zur Vermeidung von Inkompatibilitäten kann der Kern auch aus mehreren Schichten bestehen. Desgleichen kann auch die Drageehülle zur Erzielung eines Depoteffekts aus mehreren Schichten bestehen, wobei die oben bei den Tabletten erwähnten Hilfsstoffe verwendet werden können.

Säfte der erfindungsgemäßen Wirkstoffe bzw. Wirkstoffkombinationen können zusätzlich noch ein Süßungsmittel, wie Saccharin, Cyclamat, Glycerin oder Zucker, sowie ein geschmacksverbesserndes Mittel, z.B. Aromastoffe, wie Vanillin oder Orangenextrakt, enthalten. Sie können außerdem Suspendierhilfsstoffe oder Dickungsmittel, wie Natriumcarboxymethyl-cellulose,

Netzmittel, beispielsweise Kondensationsprodukte von Fettalkoholen mit Ethylenoxid, oder Schutzstoffe, wie p-Hydroxybenzoate, enthalten.

Injektionslösungen werden in üblicher Weise, z.B. unter Zusatz von Konservierungsmitteln, wie p-Hydroxybenzoaten, oder Stabilisatoren, wie Komplexonen, hergestellt und in Injektionsflaschen oder Ampullen abgefüllt.

Die Wirkstoffe bzw. Wirkstoffkombinationen enthaltenden Kapseln können beispielsweise hergestellt werden, indem man die Wirkstoffe mit inerten Trägern, wie Milchzucker oder Sorbit, mischt und in Gelatinekapseln einkapselt.

Geeignete Zäpfchen lassen sich beispielsweise durch Vermischen der dafür vorgesehenen Wirkstoffe bzw. Wirkstoffkombinationen mit üblichen Trägermitteln, wie Neutralfetten oder Polyäthylenglykol bzw. dessen Derivaten, herstellen.

Formulierungsbeispiele

1. Tabletten

| | |
|---|---|
| Verbindung nach Beispiel 3 | 10,0 mg |
| Maisstärke | 99,0 mg |
| sek. Calciumphosphat | 140,0 mg |
| Magnesiumstearat | 1,0 mg |
| | 250,0 mg |

Die Bestandteile werden in üblicher Weise zu Tabletten von 250 mg Gewicht verarbeitet.

2. Kapseln

| | |
|---|---|
| Verbindung nach Beispiel 1 | 150,0 mg |
| Maisstärke | 150,0 mg |
| | 300,0 mg |

Die fein gepulverten Komponenten werden intensiv vermischt. Jeweils 300 mg der Mischung werden in übliche Gelatinekapseln abgefüllt.

Die erfindungsgemäßen Verbindungen sind auch für die Kombination mit anderen pharmakodynamisch wirksamen Stoffen wie z.B. Diuretika, ß-Adrenolytika, Calciumantagonisten oder Tranquilizern geeignet.

Die folgenden Beispiele erläutern die Erfindung, ohne sie zu beschränken.

Herstellungsbeispiele

Beispiel 1

[1-[2,6-Dimethyl-4-(2-m-tolyloxyacetylamino)-phenoxy]-2-hydroxy-3-diethylamino-propan]hydrochlorid

5 g (0,015 mol) 1-[2,6-Dimethyl-4-(2-m-tolyloxyacetyl-amino)-phenoxy]-2,3-oxiran werden in 80 ml Ethanol gelöst, 21 ml (0,02 mol) Diethylamin zugegeben und 1,5 Stunden zum Sieden am Rückfluß erhitzt. Nach Abdestillieren des Lösungsmittels wird der Rückstand in verdünnter Salzsäure gelöst und mit Ether ausgeschüttelt. Die wäßrige Phase wird mit 20 %iger Natronlauge alkalisch gemacht, mit Methylenchlorid extrahiert und die organische Phase über Natriumsulfat getrocknet. Das Lösungsmittel wird abdestilliert und der verbleibende Rückstand über eine Kieselgelsäule (Essigester/Isopropanol/Ammoniak im Verhältnis 70 : 30 : 2) gereinigt. Die isolierte einheitliche Substanz wird in Acetonitril gelöst und durch Zugabe von etherischer Salzsäure und Ether das Hydrochlorid zur Kristallisation gebracht. Das Kristallisat wird aus Ethanol unter Zugabe von Ether umkristallisiert.
Ausbeute: 6,7 g, Fp: 159-160°C.

Beispiel 2

[1-[2,6-Dimethyl-4-(2-m-tolyloxyacetylamino)-phenoxy]-2-hydroxy-3-piperidino-propan]hydrochlorid

6 g (0,018 mol) 1-[2,6-Dimethyl-4-(2-m-tolyloxyacetyl-amino)-phenoxy]propan-2,3-oxiran werden in 100 ml Ethanol gelöst und nach Zugabe von 2 ml (0,02 mol) Piperidin zwei Stunden zum Sieden unter Rückfluß erhitzt. Das Ethanol wird abdestilliert, der Rückstand in verdünnter Salzsäure gelöst, mit Ether ausgeschüttelt und mit Natronlauge

alkalisch gemacht. Die ausfallende Base wird in Methylenchlorid aufgenommen, die organische Phase über Natriumsulfat getrocknet und das Lösungsmittel abdestilliert. Die Reinigung der Base erfolgt über eine Kieselgelsäule wie in Beispiel 1. Die Reinsubstanz wird in Acetonitril gelöst, etherische Salzsäure zugegeben, sodann Ether zugesetzt, bis Kristallisation einsetzt. Das farblose Hydrochlorid wird in Acetonitril gelöst und durch Zugabe von Ether zum Kristallisieren gebracht.
Ausbeute: 5,8 g, Fp: 189-190°C.

Beispiel 3

[1-[2,6-Dimethyl-4-(2-(2,6-dimethyl-phenoxy)-acetylamino)-phenoxy]-2-hydroxy-3-diethylamino-propan]hydrochlorid

38,8 g [1-[2,6-Dimethyl-4-(2-(2,6-dimethyl-phenoxy)-acetylamino)-phenoxy]-2,3 oxiran werden in 300 ml Ethanol gelöst und nach Zugabe von 40 ml Diethylamin zwei Stunden am Rückfluß zum Sieden erhitzt. Nach Abdestillieren des Ethanols wird der verbleibende Rückstand mit verdünnter Salzsäure angesäuert und mit Ether ausgeschüttelt. Die wäßrige Phase wird mit verdünnter Natronlauge alkalisch gemacht, die sich abscheidende Base in Ether aufgenommen und die organische Phase über Natriumsulfat getrocknet. Der Ether wird abdestilliert, der Rückstand über eine Kieselgelsäule gereinigt (Essigester/Isopropanol/Ammoniak im Verhältnis 70 : 30 : 5). Die Reinsubstanz wird in Acetonitril gelöst, etherische Salzsäure und dann Ether zugegeben, wodurch das Hydrochlorid kristallin abgeschieden wird. Dieses wird in Acetonitril gelöst und Ether bis zum Kristallisationsbeginn zugegeben. Es werden 28,6 g farbloses Kristallisat gewonnen.
Fp: 167-168°C.

In Analogie zu den Beispielen werden die in den Tabellen I bis III aufgeführten Verbindungen der Formel I synthetisiert. Die Schmelzpunkte sind, falls nicht anders bezeichnet, für die Hydrochloride angegeben. Die "viskosen Öle" sind Basen.

Die Verbindungen der Tabelle I entsprechen der Formel

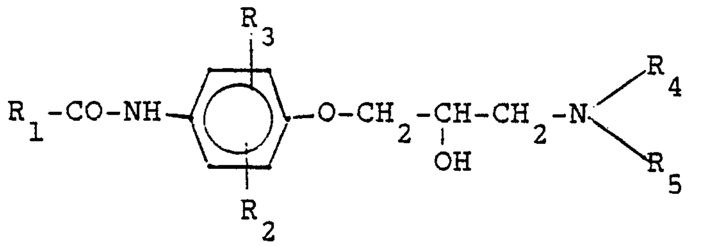

Tabelle I

| Beispiel | $R_1$ | $R_2$ | $R_3$ | $R_4$ | $R_5$ | Fp, °C |
|---|---|---|---|---|---|---|
| 4 | 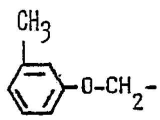 | 2-$CH_3$ | 6-$CH_3$ | $CH_3$ | $CH_3$ | 152-153 (Base) |
| 5 | " | " | " | $-CH_2-CH_2OH$ | $-CH_2-CH_2OH$ | 97-99 |
| 6 | " | " | " | $C_3H_7$ | $C_3H_7$ | 126-128 |
| 7 | " | " | " | $C_2H_5$ | $C_3H_7$ | 128-131 |
| 8 | " | " | " | iso$C_3H_7$ | iso$C_3H_7$ | 128-130 |
| 9 | " | " | " | $CH_3$ | iso$C_3H_7$ | 110-112 |
| 10 | " | " | " | $C_4H_9$ | $C_4H_9$ | 88-90 |
| 11 | " | " | " | sec$C_4H_9$ | sec$C_4H_9$ | 72-73 (Base) |
| 12 | " | " | " | $CH_3$ | n- $C_3H_7$ | 129-131 |
| 13 | " | " | " | $CH_3$ | $C_2H_5$ | 138-139 |
| 14 | " | " | " | $-CH_2-CH=CH_2$ | $-CH_2-CH=CH_2$ | 122-123 |
| 15 | " | " | " | $CH_3$ | 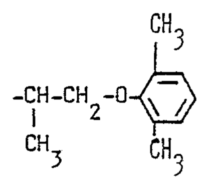 | 76-79 |
| 16 | " | " | " | $CH_3$ | 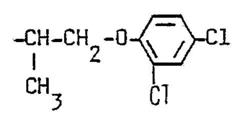 | 83-86 |
| 17 | " | " | " | $CH_3$ | 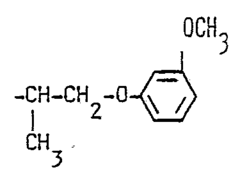 | 70-74 |
| 18 | " | " | " | $CH_3$ | 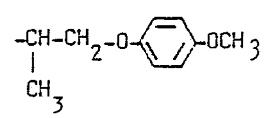 | 71-75 |
| 19 | " | " | " | $CH_3$ | 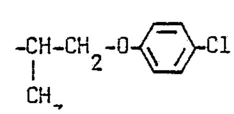 | 79-82 |

| Beispiel | $R_1$ | $R_2$ | $R_3$ | $R_4$ | $R_5$ | Fp, °C |
|---|---|---|---|---|---|---|
| 20 | 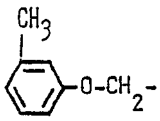 | 2-$CH_3$ | 6-$CH_3$ | H | 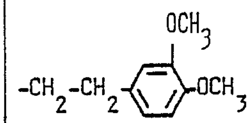 | 127-130 (Base) |
| 21 | " | " | " | $C_2H_5$ | " | 90-94 |
| 22 | " | " | " | iso$C_3H_7$ | " | 82-85 |
| 23 | " | " | " | $CH_3$ | " | 80-84 |
| 24 | 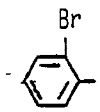 | " | " | $CH_3$ | $CH_3$ | viskoses Öl |
| 25 | " | " | " | $C_2H_5$ | $C_2H_5$ | " |
| 26 | 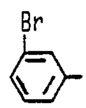 | " | " | $CH_3$ | $CH_3$ | " |
| 27 | " | " | " | $C_2H_5$ | $C_2H_5$ | " |
| 28 | 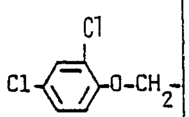 | " | " | $CH_3$ | $CH_3$ | 117-118 (Base) |
| 29 | " | " | " | $C_2H_5$ | $C_2H_5$ | 101-102 (Base) |
| 30 | " | " | " | $-CH_2-CH_2OH$ | $-CH_2-CH_2OH$ | 104-107 |
| 31 | 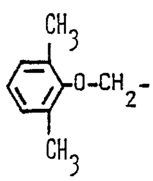 | 2-Cl | 6-Cl | $CH_3$ | $CH_3$ | 122-123 (Base) |
| 32 | " | " | " | $C_2H_5$ | $C_2H_5$ | viskoses Öl |
| 33 | " | " | " | $-CH_2-CH_2OH$ | $-CH_2-CH_2OH$ | 158-159 |
| 34 | 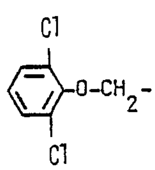 | 2-$CH_3$ | 6-$CH_3$ | $CH_3$ | $CH_3$ | 171-172 |
| 35 | " | " | " | $-CH_2-CH_2OH$ | $-CH_2-CH_2OH$ | 102-103 (Base) |

| Beispiel | $R_1$ | $R_2$ | $R_3$ | $R_4$ | $R_5$ | Fp, °C |
|---|---|---|---|---|---|---|
| 36 | 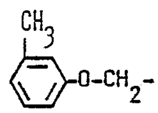 | 2-Cl | 6-Cl | $CH_3$ | $CH_3$ | 91-93 (Base) |
| 37 | " | " | " | $C_2H_5$ | $C_2H_5$ | 98-99 (Base) |
| 38 | " | " | " | $-CH_2-CH_2OH$ | $-CH_2-CH_2OH$ | 139-141 (Oxalat) |
| 39 | 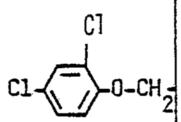 | " | " | " | " | 149-151 |
| 40 | 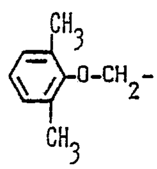 | 2-$CH_3$ | 6-$CH_3$ | $CH_3$ | $CH_3$ | 190-191 |
| 41 | " | " | " | $C_2H_5$ | $C_2H_5$ | 167-168 |
| 42 | 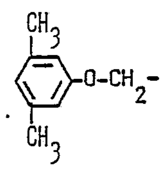 | 2-$CH_3$ | 6-$CH_3$ | $-CH_2-CH_2OH$ | $-CH_2-CH_2OH$ | 164-166 |
| 43 | " | " | " | $-CH_2-\underset{OH}{CH}-\underset{OH}{CH_2}$ | $-CH_2-\underset{OH}{CH}-CH_2OH$ | 86-89 (Base) |
| 44 | " | " | " | $CH_3$ | 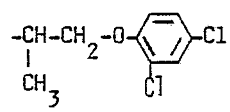 | 81-84 |
| 45 | " | " | " | $CH_3$ | 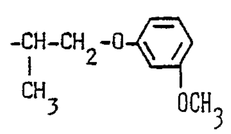 | 78-81 |
| 46 | " | " | " | $CH_3$ | 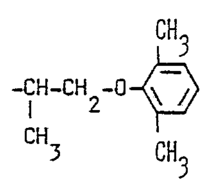 | 117-120 (Oxalat) |
| 47 | " | " | " | $CH_3$ | 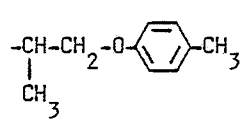 | 84-87 |
| 48 | " | " | " | $CH_3$ | 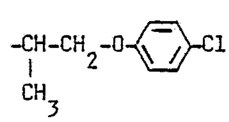 | 75-78 |

| Beispiel | $R_1$ | $R_2$ | $R_3$ | $R_4$ | $R_5$ | Fp, °C |
|---|---|---|---|---|---|---|
| 49 | 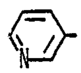 | 2-CN | H | $CH_3$ | $CH_3$ | 89-91 (Base) |
| 50 | " | " | " | $C_2H_5$ | $C_2H_5$ | 128-130 (Base) |
| 51 | 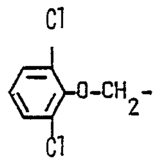 | 2-Cl | 6-Cl | $CH_3$ | $CH_3$ | 232-234 |
| 52 | " | " | " | $-CH_2-CH_2OH$ | $-CH_2-CH_2OH$ | 158-159 (Base) |
| 53 | 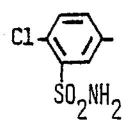 | 2-CN | " | $CH_3$ | $CH_3$ | 138-143 |
| 54 | 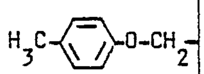 | 2-Cl | 6-Cl | $CH_3$ | $CH_3$ | 73-74 (Base) |
| 55 | " | " | " | $C_2H_5$ | $C_2H_5$ | viskoses Öl |
| 56 | " | " | " | $-CH_2-CH_2OH$ | $-CH_2-CH_2OH$ | 98-101 |
| 57 | 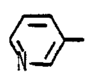 | $2-CH_3$ | $6-CH_3$ | $CH_3$ | $CH_3$ | 203-206 |
| 58 | " | " | " | $C_2H_5$ | $C_2H_5$ | 147-150 |
| 59 | 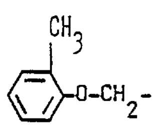 | 2-Cl | 6-Cl | $CH_3$ | $CH_3$ | 103-104 (Base) |
| 60 | " | " | " | $C_2H_5$ | $C_2H_5$ | 72-74 (Base) |
| 61 | " | " | " | $-CH_2-CH(OH)-CH_2OH$ | $-CH_2-CH(OH)-CH_2OH$ | 97-99 (Oxalat) |

| Beispiel | $R_1$ | $R_2$ | $R_3$ | $R_4$ | $R_5$ | Fp, °C |
|---|---|---|---|---|---|---|
| 62 | 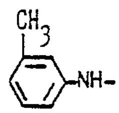 | 2-$CH_3$ | 6-$CH_3$ | $C_2H_5$ | $C_2H_5$ | 166-167 |
| 63 | " | " | " | $CH_3$ | $CH_3$ | 158-159 |
| 64 | " | " | " | $C_3H_7$ | $C_3H_7$ | 157-158 |
| 65 | 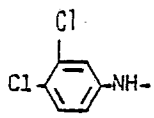 | " | " | $C_3H_7$ | $C_3H_7$ | 183-184 (Base) |
| 66 | " | " | " | $C_2H_5$ | $C_2H_5$ | 168-169 (Base) |
| 67 | 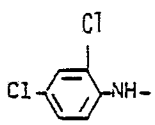 | " | " | $CH_3$ | $CH_3$ | 180-181 (Base) |
| 68 | 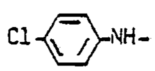 | " | " | $C_2H_5$ | $C_2H_5$ | 160-163 (Base) |
| 69 | " | " | " | $CH_3$ | $CH_3$ | 183-185 (Base) |
| 70 | " | " | " | $C_3H_7$ | $C_3H_7$ | 172-173 (Base) |
| 71 | 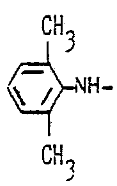 | " | " | $C_2H_5$ | $C_2H_5$ | 180-181 (Base) |
| 72 | " | " | " | $C_3H_7$ | $C_3H_7$ | 179-181 (Base) |
| 73 | " | " | " | $CH_3$ | $CH_3$ | 211-213 (Base) |

| Beispiel | $R_1$ | $R_2$ | $R_3$ | $R_4$ | $R_5$ | Fp.°C |
|---|---|---|---|---|---|---|
| 74 | 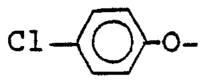 O-$CH_2$- | 2-Cl | H | $C_2H_5$ | $C_2H_5$ | 138-144 (Oxalat) |
| 75 | 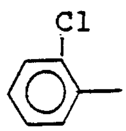 O-$CH_2$- | " | " | " | " | 155-157 |
| 76 | 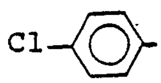 O-$CH_2$- | " | " | $CH_3$ | $CH_3$ | 149-150 (Oxalat) |
| 77 | 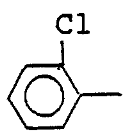 O-$CH_2$- | " | " | " | " | 190-191 (Oxalat) |

Weitere erfindungsgemäße Verbindungen der Formel

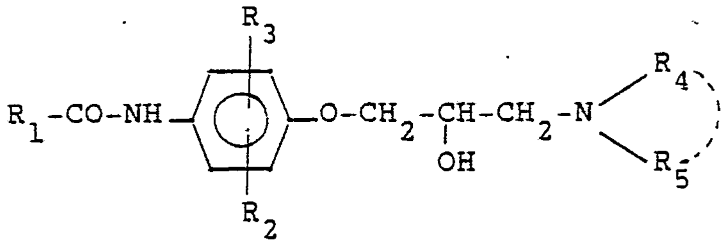

sind in der Tabelle II aufgeführt.

Tabelle II

| Bei-spiel | $R_1$ | $R_2$ | $R_3$ | 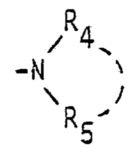 | Fp, °C |
|---|---|---|---|---|---|
| 78 | 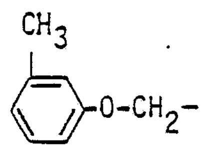 | 2-$CH_3$ | 6-$CH_3$ | 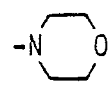 | 190-191 |
| 79 | " | " | " | 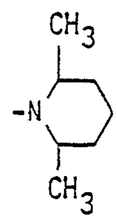 | 146-147 (Base) |
| 80 | " | " | " | 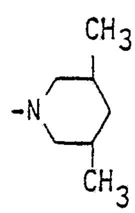 | 162-164 |
| 81 | 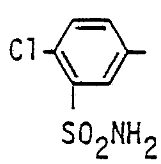 | 2-CN | H | 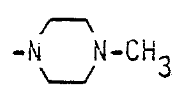 | 244-246 |

Tabelle III

Die in dieser Tabelle enthaltenen erfindungsgemäßen Verbindungen entsprechen der Formel

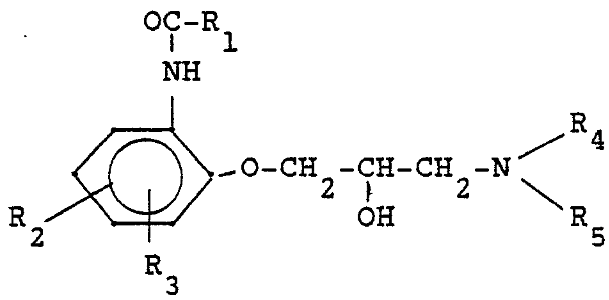

| Beispiel | $R_1$ | $R_2$ | $R_3$ | $R_4$ | $R_5$ | Fp.°C |
|---|---|---|---|---|---|---|
| 82 | 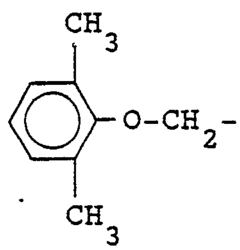 | H | H | $CH_3$ | $CH_3$ | 150-2 |
| 83 | " | H | H | i-$C_3H_7$ | H | 144-6 |
| 84 | " | H | H | $C_2H_5$ | $C_2H_5$ | 111-2 |
| 85 | " | H | H | n-$C_3H_7$ | H | 114-5 |
| 86 | 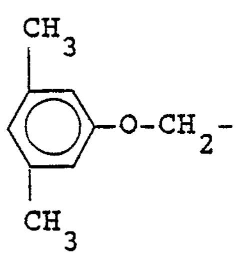 | H | H | i-$C_3H_7$ | H | 184-5 |
| 87 | " | H | H | $CH_3$ | $CH_3$ | 171-3 |
| 88 | " | H | H | n-$C_3H_7$ | H | 157-8 |

| Beispiel | $R_1$ | $R_2$ | $R_3$ | $R_4$ | $R_5$ | Fp.°C |
|---|---|---|---|---|---|---|
| 89 | 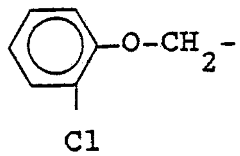 | H | H | $C_2H_5$ | $C_2H_5$ | 136-8 |
| 90 | 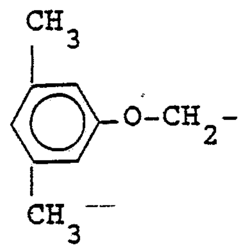 | H | H | $t-C_4H_9$ | H | 159-60 |
| 91 | 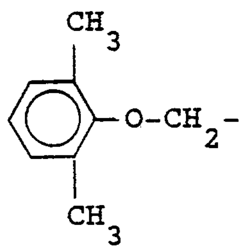 | $6-CH_3$ | H | $C_2H_5$ | $C_2H_5$ | 150-1 |
| 92 | 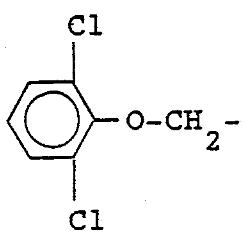 | H | H | $CH_3$ | $CH_3$ | 124-5 (Oxalat) |
| 93 | 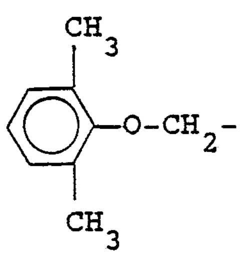 | $6-CH_3$ | H | $i-C_3H_7$ | $CH_3$ | 156-7 |
| 94 | " | $6-CH_3$ | H | $CH_3$ | $CH_3$ | 146-8 (Oxalat) |
| 95 | " | $6-CH_3$ | H | $t-C_4H_9$ | H | 169-70 |
| 96 | " | H | H | $t-C_4H_9$ | H | 180-1 |

| Beispiel | $R_1$ | $R_2$ | $R_3$ | $R_4$ | $R_5$ | Fp. °C |
|---|---|---|---|---|---|---|
| 97 | 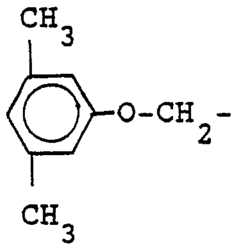 | H | H | $C_2H_5$ | $C_2H_5$ | 139-40 |
| 98 | 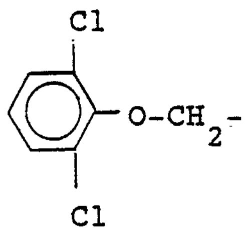 | H | H | $n-C_3H_7$ | H | 145-7 (Oxalat) |
| 99 | " | H | H | $t-C_4H_9$ | H | 157-9 |
| 100 | " | H | H | $i-C_3H_7$ | H | 132-3 |
| 101 | 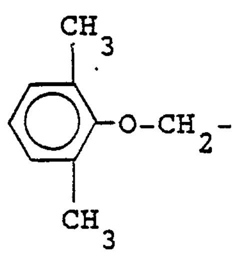 | $6-CH_3$ | H | $n-C_3H_7$ | H | 101-2 |
| 102 | 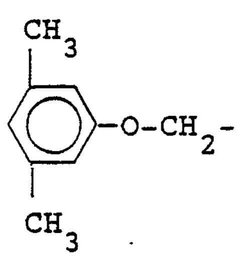 | $6-CH_3$ | H | $C_2H_5$ | $C_2H_5$ | 113-50 |
| 103 | " | $6-CH_3$ | H | $i-C_3H_7$ | H | 153-6 |
| 104 | " | $6-CH_3$ | H | $CH_3$ | $CH_3$ | 122-3 |
| 105 | " | $6-CH_3$ | H | $t-C_4H_9$ | H | 189-91 |
| 106 | " | $6-CH_3$ | H | $n-C_3H_7$ | H | 128-30 |

| Beispiel | $R_1$ | $R_2$ | $R_3$ | $R_4$ | $R_5$ | Fp.°C |
|---|---|---|---|---|---|---|
| 107 | 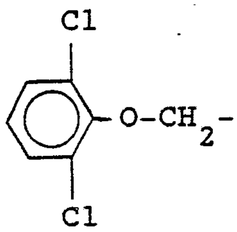 | 6-$CH_3$ | H | $C_2H_5$ | $C_2H_5$ | 146-8 |
| 108 | " | 6-$CH_3$ | H | t-$C_4H_9$ | H | 130-2 |
| 109 | " | 6-$CH_3$ | H | i-$C_3H_7$ | H | 152-4 (Oxalat) |
| 110 | " | 6-$CH_3$ | H | $CH_3$ | $CH_3$ | 132-3 (Oxalat) |
| 111 | " | 6-$CH_3$ | 6-$CH_3$ | $C_2H_5$ | $C_2H_5$ | |
| 112 | " | 3-$CH_3$ | H | $C_2H_5$ | $C_2H_5$ | |
| 113 | 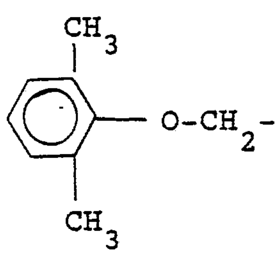 | 4-$CH_3$ | 6-$CH_3$ | $C_2H_5$ | $C_2H_5$ | |
| 114 | " | 5-$CH_3$ | H | $C_2H_5$ | $C_2H_5$ | |
| 115 | 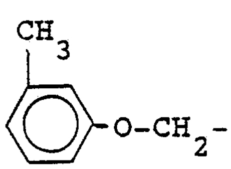 | 4-$CH_3$ | 6-$CH_3$ | $CH_3$ | n-$C_3H_7$ | |

Pharmakologische Untersuchungen

Die erfindungsgemäßen Verbindungen wurden entsprechend C. Lillie et al., Arzneimittelforschung/Drug Research 35, 301-305 (1985) untersucht.

Isolierte Meerschweinchenherzen nach Langendorff, Feuchtgewicht 1,0 - 1,2 g, Durchströmung mit Standard-Tyrode (31°C, gesättigt mit $O_2$ + 2 % $CO_2$) bei konstantem Druck (60 cm $H_2O$). Nach Zerstörung der Sinusknotenregion am rechten Vorhof elektrische ventrikuläre Reizung mit Rechteckimpulsen (1 mA, 10 msec) bei 2,5 Hz. Vorspannung 2 g, isometrische Registrierung der Kontraktionen.

Die Perfusion mit substanzhaltiger Tyrode (Konzentrationsangabe in µg/ml) erfolgte 30 min. lang.

Angegeben sind Änderungen in Prozent der Vorwerte (Δ%) der EKG-Parameter ST und QRS sowie der Kontraktionsamplitude und des Durchflusses am Ende der Perfusion mit substanzhaltiger Tyrode.

Die Ergebnisse für einige der untersuchten Verbindungen finden sich in der folgenden Tabelle.

| | | Änderungen (Δ%) nach 30 min. | | | |
|---|---|---|---|---|---|
| Verbindung | µg/ml | ST | QRS | Kontraktilität | Durchfluß |
| Beispiel 1 | 1 | + 39 | + 4 | - 31 | + 5 |
| Beispiel 12 | 1 | + 38 | + 5 | - 23 | - 2 |
| Beispiel 41 | 1 | + 31 | + 2 | - 9 | + 5 |

Wie die Werte in der Tabelle zeigen, ist in erwünschter Weise der ST-Wert deutlich erhöht, der QRS-Wert dagegen, wie auch der Durchfluß, nur wenig.

Patentansprüche

1. Verbindungen der Formel

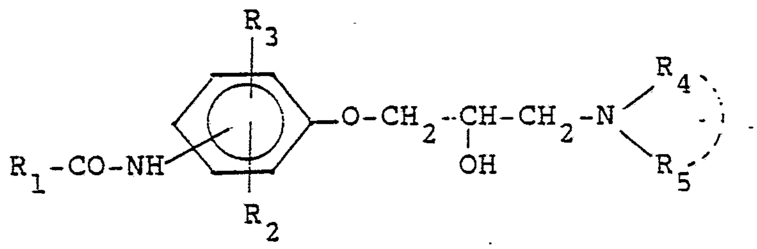

(I),

in der

$R_1$ einen Phenylrest, der gegebenenfalls durch ein oder mehrere Halogenatome, niedere Alkyl-, Alkoxy-, Alkenyl-, Alkinyl-, Alkenyloxy-, Alkinyloxy-, Cycloalkyl-, Acyl-, Acyloxy-, Alkoxycarbonyl-, Hydroxyalkyl- oder Alkoxyalkylreste oder die Sulfamoylgruppe oder die ringbildenden Gruppen $-(CH=CH)_2-$, $-O-CH_2-O-$, mit Bindung der freien Valenzen in o-Stellung zueinander, substituiert sein kann, oder einen Aryloxyalkylrest, der gegebenenfalls durch ein oder mehrere Halogenatome, niedere Alkyl-, Alkoxy-, Alkenyl-, Alkinyl-, Alkenyloxy-, Alkinyloxy, Hydroxyalkyl-, Alkoxyalkyl-, Acyl-, Acyloxy- oder Alkoxycarbonylreste sowie die ringbildende Gruppe $-(CH=CH)_2-$ oder $-OCH_2-O-$ mit Bindung der freien Valenzen in o-Stellung zueinander substituiert sein kann, oder einen Pyridylrest, oder einen Anilinorest, der durch ein oder mehrere Halogenatome oder niedere Alkylgruppen substituiert sein kann,

$R_2$ ein Wasserstoff- oder Halogenatom, eine Alkyl- oder Alkoxygruppe mit 1 bis 4 C-Atomen oder die ringbildenden Gruppen $-(CH=CH)_2-$ oder $-(CH_2)_n-$ (n = ganze Zahl von 3 bis 5) mit Bindung der freien Valenzen in o-Stellung zueinander, oder eine CN-Gruppe,

$R_3$ ein Wasserstoff- oder Halogenatom oder eine Alkylgruppe mit 1 bis 4 C-Atomen,

$R_4$ einen geraden oder verzweigten Alkylrest mit 1 bis 10 C-Atomen oder einen Hydroxyalkylrest mit 2 bis 5 C-Atomen,

$R_5$ einen geraden oder verzweigten Alkylrest mit 1 bis 10 C-Atomen oder einen Hydroxyalkylrest mit 2 bis 5 C-Atomen oder einen Phenylalkylrest oder einen Phenoxyalkylrest, wobei der aromatische Anteil durch Alkyl- oder Alkoxygruppen, durch Chlor- oder Bromatome substituiert sein kann,

$R_4$ und $R_5$ auch zusammen mit dem Stickstoffatom einen Heterocyclus bedeuten,

in Form von Racematen, reinen Enantiomeren oder Enantiomerengemischen sowie die jeweiligen Säureadditionssalze.

2. Verbindungen nach Anspruch 1, worin $R_1$ einen substituierten Phenoxymethylrest, $R_2$ und $R_3$ Halogen, Cyano oder Methyl, einer von beiden auch Wasserstoff, $R_4$ und $R_5$ niedere geradkettige oder verzweigte Alkylreste bedeuten.

3. Verbindungen nach Anspruch 1 oder 2, worin $R_1$-CO-NH sich in 4-Stellung der Phenoxygruppe befindet, $R_1$ einen ein- oder zweifach methylsubstituierten Phenoxymethylrest darstellt, $R_2$ und $R_3$ Chlor oder Methyl, $R_4$ und $R_5$ Methyl, Ethyl, n-Propyl oder i-Propyl bedeuten, wobei $R_2/R_3$ und $R_4/R_5$ jeweils gleich oder verschieden sein können.

4. [1-[2,6-Dimethyl-4-(2-m-tolyloxyacetylamino)-phenoxy]-2-hydroxy-3-diethylamino-propan]hydrochlorid und seine Säureadditionssalze.

5. [1-[2,6-Dimethyl-4-(2-m-tolyloxyacetylamino)-phenoxy]-2-hydroxy-3-piperidino-propan]hydrochlorid und seine Säureadditionssalze.

6. [1-[2,6-Dimethyl-4-(2-(2,6-dimethyl-phenoxy)-acetyl-amino)-phenoxy]-2-hydroxy-3-diethylamino-propan]-hydrochlorid und seine Säureadditionssalze.

7. 1-[2,6-Dimethyl-4-[2-(3-methylphenoxy)-acetylamino] phenoxy]-2-hydroxy-3-methyl-n-propylaminopropan und seine Säureadditionssalze.

8. Pharmazeutische Zubereitungen, gekennzeichnet durch einen Gehalt an einer Verbindung nach Anspruch 1 bis 7 neben üblichen Hilfs- und/oder Trägerstoffen.

9. Verwendung von Verbindungen nach Anspruch 1 bis 7 bei der Herstellung von pharmazeutischen Zubereitungen zur Behandlung von Herzarrhytmien, Tachykardien oder des Bluthochdrucks.

10. Verfahren zur Herstellung von Verbindungen nach Anspruch 1 bis 7, dadurch gekennzeichnet, daß man in an sich bekannter Weise eine Verbindung der Formel

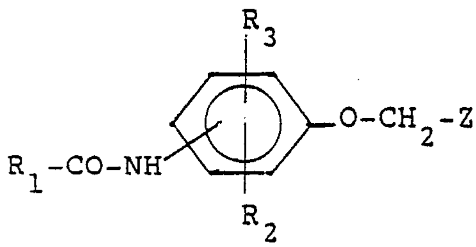

(II),

in der $R_1$, $R_2$ und $R_3$ wie oben definiert sind und Z die Gruppe $-CH - CH_2$ oder $-CHOH-CH_2-Hal$

O

(Hal = Halogen) darstellt, mit einem Amin der Formel

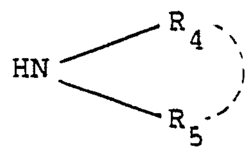

(III),

in der $R_4$ und $R_5$ die obige Bedeutung haben, umsetzt.